(19) Europäisches Patentamt
European Patent Office
Office européen des brevets

(11) Veröffentlichungsnummer: **0 263 480 A1**

(12) **EUROPÄISCHE PATENTANMELDUNG**

(21) Anmeldenummer: **87114549.6**

(22) Anmeldetag: **06.10.87**

(51) Int. Cl.⁴: **C07D 401/06**, C07D 215/26, C07D 405/06, C07D 409/06, C07D 401/14, C07D 405/14, C07D 409/14, A61K 31/47

Patentansprüche für folgenden Vertragsstaaten: GR + ES.

(30) Priorität: **06.10.86 DE 3633977**

(43) Veröffentlichungstag der Anmeldung: **13.04.88 Patentblatt 88/15**

(84) Benannte Vertragsstaaten: **AT BE CH DE ES FR GB GR IT LI NL SE**

(71) Anmelder: **CASSELLA Aktiengesellschaft**
**Hanauer Landstrasse 526**
**D-6000 Frankfurt am Main 61(DE)**

(72) Erfinder: **Rüger, Wolfgang, Dr.**
**Parkstrasse 10**
**D-6233 Kelkheim(DE)**
Erfinder: **Driesen, Gerd, Dr.**
**Forststrasse 40**
**D-6200 Wiesbaden(DE)**
Erfinder: **Bohn, Helmut, Dr.**
**Kranzbergring 11**
**D-6369 Schöneck 1(DE)**
Erfinder: **Martorana, Piero, Dr.**
**Kaiser-Friedrich-Promenade 108a**
**D-6380 Bad Homburg(DE)**

(74) Vertreter: **Urbach, Hans-Georg, Dr. et al**
**Hanauer Landstrasse 526**
**D-6000 Frankfurt am Main 61(DE)**

(54) **Tetrahydrochinolinderivate, ein Verfahren zu ihrer herstellung, ihre Verwendung und sie enthaltende pharmazeutische Zubereitungen.**

(57) Derivate des Tetrahydrochinolins der Formel I

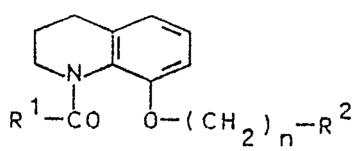

(I)

und deren Säureadditionssalze, wobei
n 2, 3, 4, 5 oder 6,
$R^1$ z.B. Alkyl, Phenyl, oder Pyridyl,
$R^2$ den Rest

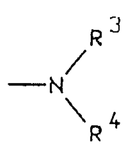

oder

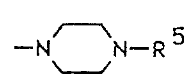

,

EP 0 263 480 A1

Xerox Copy Centre

$R^3$ und $R^4$ z.B. unabhängig voneinander Wasserstoff, Alkyl, Cycloalkyl,
$R^5$ z.B. Wasserstoff, Alkyl oder Phenyl,
bedeuten, besitzen wertvolle pharmakologische Eigenschaften.

## Tetrahydrochinolinderivate, ein Verfahren zu ihrer Herstellung, ihre Verwendung und sie enthaltende pharmazeutische Zubereitungen

Die Erfindung betrifft Tetrahydrochinolinderivate, ein Verfahren zur ihrer Herstellung, ihre Verwendung als Heilmittel sowie sie enthaltende pharmazeutische Zubereitungen.

Die vorliegende Erfindung betrifft Derivate des Tetrahydrochinolins der allgemeinen Formel I

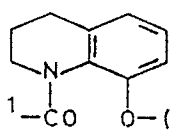

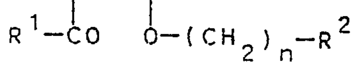

(I)

und deren Säureadditionssalze, wobei

n 2, 3, 4, 5 oder 6,

$R^1$ Alkyl mit 1 bis 5 C-Atomen, Phenyl, durch Alkyl mit 1 bis 3 C-Atomen, Alkoxy mit 1 bis 3 C-Atomen, Halogen oder Trifluormethyl mono-, di-oder trisubstituiertes Phenyl, Thienyl, Furyl, Pyrrolyl oder Pyridyl,

$R^2$ den Rest

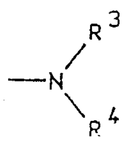 oder 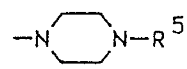 ,

$R^3$ und $R^4$ unabhängig voneinander Wasserstoff, Alkyl mit 1 bis 4 C-Atomen, Cycloalkyl mit 3 bis 8 C-Atomen, oder zusammen mit dem Stickstoffatom, an das sie gebunden sind, 1-Piperidinyl, 4-Morpholinyl, 1-Imidazolidinyl oder 1-Pyrrolidinyl,

$R^5$ Wasserstoff, Alkyl mit 1 bis 5 C-Atomen, Phenyl, Phenylalkyl mit 1 bis 4 C-Atomen im Alkylrest, Phenyloxyalkyl mit 1 bis 4 C-Atomen im Alkylrest, wobei vorhandene Phenylkerne jeweils mit Alkyl mit 1 bis 3 C-Atomen, Alkoxy mit 1 bis 3 C-Atomen, Halogen oder Trifluormethyl mono-, di-oder trisubstituiert sein können, oder Thienyl, Furyl, Pyrrolyl, Pyridinyl, Pyrimidinyl oder Triazinyl

bedeuten.

Alkylreste können, auch wenn sie in Verbindung mit anderen Resten stehen, geradkettig oder verzweigt sein. Halogensubstituenten sind vorzugsweise Chlor-oder Fluorsubstituenten. Beispiele für $R^1$ stehende Alkylreste sind: Methyl, Ethyl, n-Propyl, iso-Propyl, n-Butyl, iso-Butyl, sec.-Butyl, tert.-Butyl, n-Pentyl, iso-Pentyl. Substituenten für das für $R^1$ stehende Phenyl sind vor allem Alkyl-und/oder Alkoxyreste mit jeweils 1 bis 3 C-Atomen, ferner Halogen, insbesondere Fluor oder Chlor, sowie Trifluormethyl. Beispiele für geeignete, für $R^1$ stehende substituierte Phenylreste sind 2-, 3-oder 4-Methyl-, -Ethyl-, -n-Propyl-oder -iso-Propyl-phenyl, 2-, 3-oder 4-Methoxy-, -Ethoxy-, -n-Propoxy-phenyl, 3,5-Dimethylphenyl, 3,4,5-Trimethyl-phenyl, 3,5-Dimethoxy-phenyl, 3,4,5-Trimethoxy-phenyl, 2-, 3-oder 4-Chlor-oder -Fluor-phenyl, 2-, 3-oder 4-Trifluormethyl-phenyl. Der für $R^1$ stehende substituierte Phenylrest besitzt insbesondere einen Alkyl-, Halogen-oder Trifluormethyl-Substituenten oder einen, zwei oder drei Alkoxysubstituenten. Für $R^1$ kommen ferner Thienyl, Furyl, Pyrrolyl, vorzugsweise Pyridyl in Betracht.

Die für $R^3$ oder $R^4$ stehenden Alkylreste sind vorzugsweise verzweigt. Geeignete Alkylreste sind z.B. Methyl, Ethyl, n-Propyl, n-Butyl, sec.-Butyl, vorzugsweise iso-Propyl, iso-Butyl, tert.-Butyl. Beispiele für die für $R^3$ oder $R^4$ stehenden Cycloalkylreste sind Cyclopropyl, Cyclobutyl, Cycloheptyl, Cyclooctyl, vorzugsweise Cyclopentyl und Cyclohexyl.

In den für $R^5$ stehenden substituierten Phenyl-, Phenylalkyl-und Phenyloxyalkylresten kann der Phenylrest einen, zwei oder drei Substituenten besitzen. Beispiele für geeignete substituierte Phenylreste sind: 2-, 3-oder 4-Methyl-, -Ethyl-, -n-Propyl-oder -iso-Propyl-phenyl, 2-, 3-oder 4-Methoxy-, -Ethoxy-, -n-Propoxy-phenyl, 3,5-Dimethoxyphenyl, 3,4,5-Trimethoxyphenyl, 2-, 3-oder 4-Chlor-oder -Fluor-phenyl, 2-, 3-oder 4-Trifluormethylphenyl. Der substituierte Phenylrest in den für $R^5$ stehenden Phenyl-, Phenylalkyl-und Phenyloxyalkylresten besitzt insbesondere einen Alkyl-, Halogen-oder Trifluormethylsubstituenten oder einen, zwei oder drei Alkoxysubstituenten. In den für $R^5$ stehenden Phenylalkyl-und Phenyloxyalkylresten umfaßt die Alkylkette oder Phenyloxyalkylkette ein bis vier C-Atome, insbesondere zwei oder drei C-Atome.

Bevorzugt werden Verbindungen der allgemeinen Formel I bzw. deren Säureadditionssalze, bei denen n 2, 3 oder 4, $R^1$ Pyridyl und $R^2$ ein Rest $-NR^3R^4$ oder ein Rest

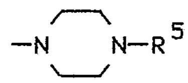

ist, wobei $R^3$ für Wasserstoff, Methyl oder n-Propyl, $R^4$ für n-Propyl, Isopropyl, tert.-Butyl oder Cyclohexyl steht, oder wobei $R^3$ und $R^4$ zusammen mit dem Stickstoffatom, an das sie gebunden sind, 4-Morpholinyl bedeuten und wobei $R^5$ für durch Alkoxy mit 1 bis 3 C-Atomen monosubstituiertes Phenyl steht.

Besonders bevorzugt werden Verbindungen der allgemeinen Formel I bzw. deren Säureadditionssalze, bei denen n 2, 3 oder 4, $R^1$ 3-Pyridyl und $R^2$ ein Rest $-NR^3R^4$ oder ein Rest

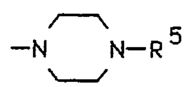

ist, wobei $R^3$ für Methyl oder n-Propyl, $R^4$ für n-Propyl oder Cyclohexyl steht, oder wobei $R^3$ und $R^4$ zusammen mit dem Stickstoffatom, an das sie gebunden sind, 4-Morpholinyl bedeuten und wobei $R^5$ für durch Methoxy monosubstituiertes Phenyl steht.

Ganz besonders bevorzugt sind 8-(2-(N-Cyclohexyl-N-methyl-amino)ethoxy)-1-nicotinoyl-1,2,3,4-tetrahydrochinolin, 8-(3-(4-(2-Methoxyphenyl)-piperazin-1-yl)propoxy)-1-nicotinoyl-1,2,3,4-tetrahydrochinolin und 8-(4-N,N-Dipropylamino-butoxy)-1-nicotinoyl-1,2,3,4-tetrahydrochinolin bzw. deren Säureadditionssalze.

Die vorliegende Erfindung betrifft weiterhin ein Verfahren zur Herstellung von Verbindungen der Formel I, dadurch gekennzeichnet, daß man

a) eine Verbindung der allgemeinen Formel II

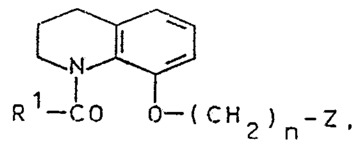

(II)

in der $R^1$ und n die genannten Bedeutungen besitzen und in der Z eine nucleophil austauschbare Gruppe bedeutet, mit einem Amin der allgemeinen Formel III

$R^2$-H (III),

in der $R^2$ die angegebene Bedeutung besitzt, in an sich bekannter Weise umsetzt und die erhaltene Verbindung gegebenenfalls mit einer Säure in ein Säureadditionssalz überführt, oder daß man

b) eine Verbindung der allgemeinen Formel IV

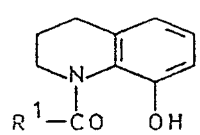

(IV),

in der $R^1$ die angegebene Bedeutung besitzt, mit einer Verbindung der allgemeinen Formel V

$R^2-(CH_2)_n-Y$ (V),

in der $R^2$ und n die angegebenen Bedeutungen besitzen und in der Y eine nucleophil austauschbare Gruppe bedeutet, in an sich bekannter Weise umsetzt und die erhaltene Verbindung gegebenenfalls mit einer Säure in ein Säureadditionssalz überführt.

Als nucleophil austauschbare Gruppen Z bzw. Y in den Ausgangsprodukten der allgemeinen Formeln II und V kommen insbesondere ein Chlor-, Brom-oder Iod-Substituent oder ein Sulfonsäurerest, vorzugsweise ein Methylsulfonyl-, Benzolsulfonyl-, p-Toluolsulfonyl-oder Trifluormethansulfonyl-rest in Betracht.

Die Umsetzung der Verbindung der allgemeinen Formel II mit einem Amin der allgemeinen Formel III wird unter den an sich bekannten Bedingungen einer nucleophilen Substitution durchgeführt. Die Umsetzung erfolgt dabei in einem geeigneten organischen Lösungsmittel ohne oder vorzugsweise in Gegenwart einer Hilfsbase zum Abfangen der sich bildenden Säure, vorzugsweise in Gegenwart von Kaliumcarbonat, Natriumcarbonat, Triethylamin, Pyridin, 1,5-Diazabicyclo(5,4,0)undec-5-en oder 1,5-Diazabicyclo(4,3,0)non-5-en, sowie in oder ohne Gegenwart eines Alkalihalogenids, vorzugsweise Natriumiodid oder Kaliumiodid, bei einer Temperatur zwischen 0 und 160°C, vorzugsweise zwischen 20 und 120°C.

Geeignete organische Lösungsmittel sind z.B. Kohlenwasserstoffe, vorzugsweise Toluol und vorzugsweise polare organische Lösungsmittel, so z.B. niedere Alkohole, vorzugsweise Methanol, Ethanol, Propanol oder Isopropanol, oder niedere Ketone, vorzugsweise Aceton, Methylethylketon oder Methylisobutylketon, oder Acetonitril, Dimethylformamid, Dimethylsulfoxid oder Sulfolan.

Die Umsetzung der Verbindung der allgemeinen Formel IV mit der Verbindung der allgemeinen Formel V wird entweder in einem polaren aprotischen Lösungsmittel, wie Acetonitril, Tetrahydrofuran, Dimethylsulfoxid, Dimethylform amid, Sulfolan oder N-Methylpyrrolidon, in Gegenwart einer starken Base, wie Natriumhydrid, Kaliumhydrid, Natriumamid, Lithiumdiisopropylamid, Butyllithium, Kalium-tert.butanolat oder Lithiumhexamethyldisilazid, vorzugsweise in Dimethylformamid oder Dimethylsulfoxid in Gegenwart von Natriumhydrid, Kalium-tert.butanolat oder Natriumamid, bei einer Temperatur zwischen -40 bis +100°C, vorzugsweise zwischen -20 bis +50°C, oder in einem protischen oder aprotischen polaren organischen Lösungsmittel, wie einem niederen Alkohol, beispielsweise Methanol, Ethanol, Isopropanol, oder einem niederen Keton, vorzugsweise Aceton, Methylethylketon oder Methylisobutylketon, oder in Dimethylformamid, in Gegenwart einer schwachen bis mittelstarken Base, wie einem Alkali-oder Erdalkalimetallhydroxid oder -carbonat, vorzugsweise Natriumcarbonat oder Kaliumcarbonat, oder einem Amin, wie beispielsweise Triethylamin, Pyridin, N-Ethyldiisopropyllamin, 1,5-Diazabicyclo(5,4,0)undec-5-en oder 1,5-Diazabicyclo(4.3.0)non-5-en, bei einer Temperatur zwischen 0 und 160°C, vorzugsweise zwischen 20 und 120°C, durchgeführt.

Zur Bildung von Säureadditionssalzen mit den Verbindungen der allgemeinen Formel I sind anorganische und organische Säuren geeignet. Geeignete Säuren sind beispielsweise Chlorwasserstoff, Bromwasserstoff, Naphthalindisulfonsäure(1,5), Phosphor-, Salpeter, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Äpfel-, Sulfamin-, Phenylpropion-, Glucon-, Ascorbin-, Isonicotin-, Nicotin-, Methansulfon-, p-Toluolsulfon-, Citronen- oder Adipinsäure. Pharmazeutisch annehmbare Säureadditionssalze werden bevorzugt. Die Säureadditionsalze können wie üblich durch Vereinigung der Komponenten, zweckmäßigerweise in einem geeigneten Verdünnungs-bzw. Dispergiermittel, erhalten werden.

Die Ausgangsverbindungen der allgemeinen Formel II können durch Umsetzung von Verbindungen der allgemeinen Formel IV mit Verbindungen der allgemeinen Formel VI

$Y-(CH_2)_n-Z$ (VI)

wobei n, Y und Z die bereits genannten Bedeutungen besitzen, unter den Bedingungen einer nucleophilen Substitution, wie sie unter der Verfahrensvariante b) beschrieben sind, hergestellt werden. Das dabei entstandene Reaktionsprodukt der Formel II kann zu seiner weiteren Umsetzung mit der Verbindung III isoliert werden, es kann aber auch ohne Isolierung direkt weiter umgesetzt werden.

Die Herstellung der Verbindungen der allgemeinen Formel IV ist bekannt und z.B. in der Europäischen Patentschrift 0 025 864 beschrieben.

Die Verbindungen der allgemeinen Formeln III, V und VI sind im Handel erhältlich, literaturbekannt oder nach bekannten einfachen Verfahren aus käuflichen Ausgangsmaterialien zugänglich.

Die erfindungsgemäßen Verbindungen der allgemeinen Formel I und deren pharmazeutisch annehmbare Säureadditionssalze besitzen wertvolle pharmazeutische Eigenschaften. Insbesondere besitzen sie blutdrucksenkende und vor allem antiarrhythmische Wirkungen. Sie sind daher z.B. für die Behandlung von Erkrankungen des Herz-Kreislauf-Systems geeignet, wie insbesondere zur Behandlung von Herzarrhythmien, ferner zur Behandlung von Hypertonie. Die erfindungsgemäßen Tetrahydrochinoline können daher am Menschen für sich allein, in Mischungen untereinander oder in pharmazeutischen Zubereitungen verabreicht werden, die als aktiven Bestandteil eine wirksame Dosis mindestens eines erfindungsgemäßen Tetrahydrochinolins oder eines pharmazeutisch annehmbaren Säureadditionssal zes davon neben üblichen pharmazeutisch einwandfreien Träger-und Zusatzstoffen enthalten.

Die erfindungsgemäßen Verbindungen der Formel I und ihre pharmazeutisch annehmbaren Säureadditionssalze sind innerhalb eines breiten Dosisbereichs wirksam. Die Höhe der verarbreichten Dosis ist abhängig von der Art der gewünschten Behandlung, von der Verabreichungsweise, vom Zustand, vom Typ und von der Größe des behandelten Patienten. Bei oraler Dosierung werden befriedigende Ergebnisse mit Dosen von ab 0,01 mg, vorzugsweise ab 0,1 mg, und bis zu 100 mg, vorzugsweise bis zu 20 mg, einer

Verbindung der Formel I pro kg Körpergewicht erreicht. Beim Menschen variiert die tägliche Dosis zwischen 1 und 1200 mg, wobei Einzeldosen von 0,5 bis 400 mg vorzugsweise ein-bis dreimal täglich gegeben werden können. Für intravenöse und intramuskuläre Anwendung beträgt die Dosis 0,1 bis 400 mg täglich.

Die Verbindungen der vorliegenden Erfindung und ihre pharmazeutisch annehmbaren Säureadditionssalze können zur Herstellung von pharmazeutischen Präparaten verwendet werden, die eine wirksame Menge der Aktivsubstanz zusammen mit pharmazeutisch zulässigen Trägerstoffen und gegebenenfalls Zusatzstoffen enthalten und die sich zur enteralen und parenteralen Verabreichung eignen. Vorzugsweise verwendet werden Tabletten oder Gelatinekapseln, die den Wirkstoff zusammen mit Verdünnungsmitteln oder Trägerstoffen, z.B. Lactose, Dextrose, Rohrzucker, Mannitol, Sorbitol, Cellulose und/oder Glycin, und Gleitmitteln, wie Kieselerde, Talk, Stearinsäure oder deren Salze wie Magnesium-oder Calciumstearat, und/oder Polyethylenglykol enthalten. Tabletten enthalten gegebenenfalls Bindemittel wie Magnesiumaluminiumsilicat, Stärke, Gelatine, Traganth, Methylcellulose, Natriumcarboxymethyl cellulose und/oder Polyvinylpyrrolidon und, falls benötigt, Farbstoffe, Geschmackstoffe, Süßmittel und andere Hilfs-bzw. Zusatzstoffe. Injizierbare Lösungen sind vorzugsweise isotonische wäßrige Lösungen oder Suspensionen, die sterilisiert sein können und Zusatz-bzw. Hilfsstoffe wie Konservier-, Stabilisierungs-, Netz-und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z.B. mittels konventioneller Misch-, Granulier-und Dragierverfahren hergestellt und enthalten 0,1 bis etwa 75 %, bevorzugt etwa 1 bis etwa 50 %, des Wirkstoffs.

Die im Anschluß folgenden Beispiele sollen die Erfindung erläutern, ohne sie auf diese Beispiele zu begrenzen.

Beispiel 1:

8-(3-(N-Isopropylamino)propoxy)-1-nicotinoyl-1,2,3,4-tetrahydrochinolin-oxalat

18,4 g (50,0 mmol) 8-(3-Chlorpropoxy)-1-nicotinoyl-1,2,3,4-tetrahydrochinolin-hydrochlorid werden in 800 ml Dimethylformamid gelöst, mit 8,3 g (50,0 mmol) Kaliumiodid und 148 g (2,50 mol) Isopropylamin versetzt und 3 Stunden bei 80°C gerührt. Danach wird die Reaktionslösung auf 3 l Eiswasser gegeben und mehrfach mit Methylenchlorid extrahiert. Die vereinigten Extrakte werden mehrfach mit Wasser gewaschen, getrocknet und am Rotationsverdampfer eingeengt. Es verbleiben 13,2 g (74,8 %) des öligen Produktes als Rückstand.

Zur Überführung in ein kristallines Derivat wird das Rohprodukt in 40 ml Aceton gelöst, tropfenweise mit einer Lösung von 3,37 g Oxalsäure in 30 ml Aceton versetzt und zwei Stunden nachgerührt. Der Niederschlag wird abgesaugt und im Vakuum getrocknet. Man erhält 10,8 g des 8-(3-(N-Isopropylamino)propoxy)-1-nicotinoyl-1,2,3,4-tetrahydrochinolin-monooxalats vom Schmelzpunkt 213-220°C. Durch Umkristallisation aus Isopropanol läßt sich analysenreine Substanz vom Schmelzpunkt 215-219°C gewinnen.

Das als Ausgangsprodukt benutzte 8-(3-Chlorpropoxy)-1-nicotinoyl-1,2,3,4-tetrahydrochinolin-hydrochlorid kann wie folgt hergestellt werden:

In eine Lösung von 35,6 g (0,14 mol) 8-Hydroxy-1-nicotinoyl-1,2,3,4-tetrahydrochinolin in 200 ml wasserfreiem Dimethylformamid wird portionsweise 7,40 g (0,154 mol) einer 50%igen Natriumhydrid-Dispersion eingetragen und 40 Minuten bei 40 - 50°C nachgerührt. Die entstandene Reaktionslösung wird in einen Tropftrichter überführt und bei 0°C zu einer Lösung von 66,0 g (0,52 mol) 1-Brom-3-chlorpropan in 100 ml wasserfreiem Dimethylformamid getropft. Anschließend wird noch 1 Stunde bei 0°C und 1 Stunde bei Raumtemperatur nachgerührt, filtriert, das Lösungsmittel und überschüssiges 1-Brom-3-chlorpropan im Ölpumpenvakuum abdestilliert. Man nimmt den Rückstand im Methylenchlorid auf, extrahiert mehrfach mit 1 N Natronlauge, trocknet und engt ein.

Das ölige Rohrprodukt wird durch Überführung in sein Hydrochlorid wie folgt weiter gereinigt: Man nimmt in Ethylacetat auf, versetzt langsam mit einer gesättigten Lösung von Chlorwasserstoff in Ethylacetat, rührt 2 Stunden bei Raumtemperatur nach, saugt den Niederschlag ab, wäscht ihn mit Ethylacetat und trocknet ihn im Vakuum. Ausbeute: 37,9 g (73,7 %) (8-(3-Chlorpropoxy)-1-nicotinoyl-1,2,3,4-tetrahydrochinolin-hydrochlorid.

Durch Umkristallisation aus Isopropanol erhält man analysenreines Produkt vom Schmelzpunkt 162-166°C.

Beispiel 2:

8-(3-(4-(2-Methoxyphenyl)piperazin-1-yl)propoxy)-1-nicotinoyl-1,2,3,4-tetrahydrochinolin-trihydrochlorid.

a) Nach Verfahrensvariante a) :
3,50 g (9,53 mmol) 8-(3-Chlorpropoxy)-1-nicotinoyl-1,2,3,4-tetrahydrochinolin-hydrochlorid, 2,74 g (11,4 mmol) 1-(2-Methoxyphenyl)piperazin, 2,88 g (28,6 mmol) Triethylamin und 1,58 g (9,53 mmol) Kaliumiodid werden in 50 ml wasserfreiem Dimethylformamid vereinigt und 18 Stunden bei 80°C gerührt. Nach dem Abkühlen wird die Reaktionslösung auf 300 ml Wasser gegeben und zweimal mit Methylenchlorid extrahiert. Die vereinigten organischen Phasen werden anschließend mit 2 N Salzsäure extrahiert, die sauren Extrakte durch Zugabe von konzentrierter Natronlauge basisch gestellt und erneut mit Methylenchlorid extrahiert. Diese Methylenchlorid-Extrakte werden getrocknet, eingeengt und das Rohprodukt durch Säulenchromatographie an Kieselgel (Laufmittel Toluol:Ethanol 9:1) weiter gereinigt. Man erhält 4,2 g (90%) öliges 8-(3-(4-(2-Methoxyphenyl)piperazin-1-yl)propoxy)-1-nicotinoyl-1,2,3,4-tetrahydrochinolin. Durch Lösen in 30 ml Aceton, Zugabe von 30 ml einer gesättigten Lösung von Chlorwasserstoff in Diethylether und Verreiben mit Diethylether erhält man hieraus 2,4 g analysenreines 8-(3-(4-(2-Methoxyphenyl)piperazin-1-yl)-propoxy)-1-nicotinoyl-1,2,3,4-tetrahydrochinolin-trihydrochlorid vom Schmelzpunkt 186-190°C (Zers.).

b) Nach Verfahrensvariante b):
In eine Lösung von 2,54 g (10,0 mmol) 8-Hydroxy-1-nicotinoyl-1,2,3,4-tetrahydrochinolin in 20 ml wasserfreiem Dimethylformamid werden portionsweise 0,53 g (11,0 mmol) einer 50%igen Natriumhydrid-Dispersion eingetragen und 30 Minuten bei 40 - 50°C nachgerührt. Nach dem Abkühlen auf Raumtemperatur wird eine Lösung von 4,03 g (15,0 mmol) 1-(3-Chlorpropyl)-4-(2-methoxyphenyl)piperazin in 20 ml wasserfreiem Dimethylformamid zugetropft und 16 Stunden bei Raumtemperatur nachgerührt. Die Reaktionslösung wird auf 150 ml Eiswasser gegeben, mit Methylenchlorid extrahiert, die Extrakte mehrfach mit Wasser gewaschen, getrocknet und eingeengt. Das Rohprodukt wird durch Säulenchromatographie an Kieselgel (Laufmittel Toluol:Ethanol 9:1) weiter gereinigt und, wie unter Beispiel 2a) angegeben, in das Dihydrochlorid überführt. Das so erhaltene Produkt ist mit dem unter 2a) beschriebenen Produkt identisch.

Entsprechend den Beispielen 1 und 2 wurden die folgenden Verbindungen hergestellt:

Beispiel 3:

8-(3-(4-(2-(3-Trifluormethyl-phenoxy)ethyl)-piperazin-1-yl)propoxy)-1-nicotinoyl-1,2,3,4-tetrahydrochinolinoxalat,

Schmelzpunkt 216-220°C (Zers.).

Beispiel 4:

8-(2-(N-Isopropylamino)ethoxy)-1-nicotinoyl-1,2,3,4-tetrahydrochinolin-oxalat.

Schmelzpunkt 153-158°C (Zers.).

Beispiel 5:

8-(2-(N-tert.Butylamino)ethoxy)-1-nicotinoyl-1,2,3,4-tetrahydrochinolin-oxalat.

Schmelzpunkt 154-160°C (Zers.).

Beispiel 6:

8-(2-(N-Cyclohexylamino)ethoxy)-1-nicotinoyl-1,2,3,4-tetrahydrochinolin.

Schmelzpunkt 216-218°C.

Beispiel 7:

8-(2-(N-Cyclohexyl-N-methyl-amino)ethoxy)-1-nicotinoyl-1,2,3,4-tetrahydrochinolin-oxalat.

Schmelzpunkt 130-131 °C (Zers.).

Beispiel 8:

8-(4-(N-Isopropylamino)butoxy-1-nicotinoyl-1,2,3,4-tetrahydrochinolin-oxalat.

Schmelzpunkt 160-163°C.

Beispiel 8:

8-(4-(N-Methyl-piperazin-1-yl)butoxy)-1-nicotinoyl-1,2,3,4-tetrahydrochinolin-dioxalat.

Schmelzpunkt 203-204°C (Zers.).

Beispiel 10:

8-(4-Morpholino-butoxy)-1-nicotinoyl-1,2,3,4-tetrahydrochinolin-oxalat.

Schmelzpunkt 175-175°C (Zers.).

Beispiel 11:

8-(4-N,N-Dipropylamino-butoxy)-1-nicotinoyl-1,2,3,4-tetrahydrochinolin-oxalat.

Schmelzpunkt 128-134°C.

Beispiel 12

8-(3-(4-(2-(3,4-Dimethoxyphenyl)ethyl)-piperazin-1-yl) propoxy)-1-picolinoyl-1,2,3,4-tetrahydrochinolin-trihydrochlorid.

Schmelzpunkt: 198°C

Beispiel 13

8-(3-(4-(2-(3,4-Dimethoxyphenyl)ethyl)-piperazin-1-yl) propoxy)-1-(3,4,5-trimethoxybenzoyl)-1,2,3,4-tetrahydrochinolin-fumerat.

Schmelzpunkt: 166°C

Beispiel 14

8-(3-(4-(2-(3,4-Dimethoxyphenyl)ethyl)-piperazin-1-yl) propoxy)-1-nicotinoyl-1,2,3,4-tetrahydrochinolin.

öl

Beispiel 15

8-(3-(N-Cyclohexyl-N-ethyl-amino)propoxy-1-nicotinoyl-1,2,3,4-tetrahydrochinolin-dihydrochlorid.

Schmelzpunkt: 90°C (Zers.)

Beispiel 16

8-(3-(N,N-Diisopropylamino)propoxy-1-nicotinoyl-1,2,3,4-tetrahydrochinolin-fumarat

Schmelzpunkt: 193°C

Beispiel 17

8-(3-(N-Benzyl-N-methyl-amino)propoxy-1-nicotinoyl-1,2,3,4-tetrahydrochinolin-dihydrochlorid.

Schmelzpunkt: 75°C (Zers.)

Die antiarrhytmische Wirkung der erfindungsgemäßen Verbindungen wurde durch Bestimmung von Kontraktionskraft und Refraktärzeit am isolierten linken Meerschweinchenvorhof geprüft. Dazu wurden Meerschweinchen durch Genickschlag getötet, das Herz rasch entfernt, die Vorhöfe freipräpariert und in einem thermostatisierten VierfachOrganbad bei einer Vorspannung von 1 g montiert. Die Kontraktionskraft der elektrisch stimulierten Vorhöfe (Frequenz 120/min, Spannung ca. 15 V, Impulsbreite 1 msec) wurde isometrisch mit Kraftaufnehmern erfaßt und das verstärkte Signal kontinuierlich aufgezeichnet.

Zur Messung der Refraktärzeit diente ein mikroprozessorgesteuerter Doppelreizgenerator, der alle 2 min 30 sec einen Meßzyklus auslöste. Dabei wurden in den Grundrhythmus Extrareize eingefügt, deren Verzögerung von 70 msec beginnend in 5 msec Schritten automatisch solange vergrößert wurde, bis eine Zunahme der Kontraktionsamplitude um ca. 25 % eintrat. Der dabei erreichte Verzögerungswert wurde als Refraktärzeit auf einem angeschlossenen Datendrucker ausgedruckt.

Die Substanzgabe erfolgte kumulativ, wobei jede Dosis 15 bis 20 min einwirkte. Da sich die Substanzeffekte gegen Ende die Answertung jeweils kurz vor Gabe der nächsthöheren Dosis. Pro Substanz wurden 4 Vorhöfe verwendet, die jeweils nur eine Substanz erhielten.

Die folgende Tabelle zeigt, daß die erfindungsgemäßen Verbindungen dem nächstliegenden Stand der Technik, 8-(3-isopropylamino-2-hydroxy-propoxy)1-nicotinoyl-1,2,3,4-tetrahydrochinolin (EP 0 025 864) deutlich überlegen sind. Die Refraktärzeiten sind bei geringerer Abnahme der Kontraktionskraft deutlich länger.

| | µM | 10 | 30 | 100 | 300 |
|---|---|---|---|---|---|
| 8-(2-(N-Cyclohexyl-N-methylamino)ethoxy)-1-nicotinoyl-1,2,3,4-tetrahydrochinolin-oxalat | Δ % Refraktärzeit | 35 | 97 | | |
| | Δ % Kraft | -21 | -30 | | |
| 8-(3-(4-(2-Methoxyphenyl)-piperazin-1-yl)propoxy)-1-nicotinoyl-1,2,3,4-tetrahydrochinolin-trihydrochlorid | Δ % Refraktärzeit | 34 | 76 | | |
| | Δ % Kraft | 0 | -7 | | |
| 8-(4-N,N-Dipropylamino-butoxy)-1-nicotinoyl-1,2,3,4-tetrahydrochinolin-oxalat | Δ % Refraktärzeit | 35 | 82 | 99 | |
| | Δ % Kraft | 6 | -6 | -28 | |
| 8-(4-Morpholino-butoxy)-1-nicotinoyl-1,2,3,4-tetrahydrochinolin-oxalat | Δ % Refraktärzeit | 23 | 37 | 60 | 82 |
| | Δ % Kraft | 11 | 13 | 10 | -4 |
| 8-(3-Isopropylamino-2-hydroxypropoxy)-1-nicotinoyl-1,2,3,4-tetrahydrochinolin (Vergleichssubstanz - EP 0 025 864) Beispiel 1) | Δ % Refraktärzeit | | 33 | 61 | |
| | Δ % Kraft | | -18 | -22 | |

Beispiel 18:

Zuckerüberzogene Pillen können nach der folgenden Rezeptur hergestellt werden:

| | |
|---|---|
| 8-(3-(4-(2-Methoxyphenyl)piperazin-1-yl)propoxy-1-nicotinoyl-1,2,3,4-tetra-hydrochinolin-trihydrochlorid | 1 mg |
| Kornstärke | 100 mg |
| Lactose | 60 mg |
| sec.Calciumphosphat | 30 mg |
| lösliche Stärke | 3 mg |
| Magnesiumstearat | 2 mg |
| kolloidale Kieselsäure | 4 mg |
| | 200 mg |

Beispiel 19:

Tabletten können nach der folgenden Rezeptur hergestellt werden:

| | |
|---|---|
| 8-(2-N-Cyclohexylamino)-ethoxy)-1-nicotinoyl-1,2,3,4-tetra-hydrochinolin | 2 mg |
| Lactose | 60 mg |
| Kornstärke | 30 mg |
| lösliche Stärke | 4 mg |
| Magnesiumstearat | 4 mg |
| | 100 mg |

**Ansprüche**

1. Derivate des Tetrahydrochinolins der allgemeinen Formel I

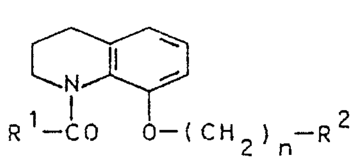

(I)

und deren Säureadditionssalze, wobei
n 2, 3, 4, 5 oder 6,
$R^1$ Alkyl mit 1 bis 5 C-Atomen, Phenyl, durch Alkyl mit 1 bis 3 C-Atomen, Alkoxy mit 1 bis 3 C-Atomen, Halogen oder Trifluormethyl mono-, di-oder trisubstituiertes Phenyl, Trienyl, Furyl, Pyrrolyl oder Pyridyl,
$R^2$ den Rest

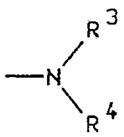

oder

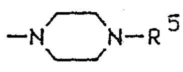,

$R^3$ und $R^4$ unabhängig voneinander Wasserstoff, Alkyl mit 1 bis 4 C-Atomen, Cycloalkyl mit 3 bis 8 C-Atomen, oder zusammen mit dem Stickstoffatom, an das sie gebunden sind, 1-Piperidinyl, 4-Morpholinyl, 1-Imidazolidinyl oder 1-Pyrrolidinyl,

$R^5$ Wasserstoff, Alkyl mit 1 bis 5 C-Atomen, Phenyl, Phenylalkyl mit 1 bis 4 C-Atomen im Alkylrest, Phenyloxyalkyl mit 1 bis 4 C-Atomen in Alkylrest, wobei vorhandene Phenylkerne jeweils mit Alkyl mit 1 bis 3 C-Atomen, Alkoxy mit 1 bis 3 C-Atomen, Halogen oder Trifluormethyl mono-, di-oder trisubstituiert sein können, oder Thienyl, Furyl, Pyrrolyl, Pyridinyl, Pyrimidinyl oder Triazinyl bedeuten.

2. Tetrahydrochinolinderivate nach Anspruch 1, dadurch gekennzeichnet, daß n 2, 3 oder 4 bedeutet.

3. Tetrahydrochinolinderivate nach Anspruch 1 und/oder 2, dadurch gekennzeichnet, daß $R^1$ Pyridyl bedeutet.

4. Tetrahydrochinolinderivate nach einem oder mehreren der Ansprüche 1 bis 3, dadurch gekennzeichnet, daß $R^2$ den Rest $-NR^3R^4$ oder den Rest

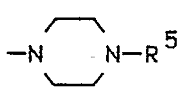

bedeutet, wobei $R^3$ für Wasserstoff, Methyl oder n-Propyl, $R^4$ für n-Propyl, Isopropyl, tert.-Butyl oder Cyclohexyl steht, oder wobei $R^3$ und $R^4$ zusammen mit dem Stickstoffatom, an das sie gebunden sind, 4-Morpholinyl bedeuten und wobei $R^5$ für durch Alkoxy mit 1 bis 3 C-Atomen monosubstituiertes Phenyl steht.

5. Verfahren zur Herstellung der in einem oder mehreren der Ansprüche 1 bis 4 angegebenen Tetrahydrochinolinderivate der Formel I, durch gekennzeichnet, daß man

a) eine Verbindung der allgemeinen Formel II

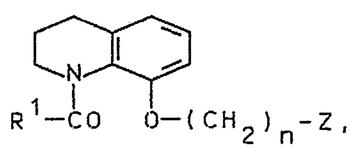

(II)

in der $R^1$ und n die genannten Bedeutungen besitzen und in der Z eine nucleophil austauschbare Gruppe bedeutet, mit einem Amin der allgemeinen Formel III

$R^2$-H (III),

in der $R^2$ die angegebene Bedeutung besitzt, in an sich bekannter Weise umsetzt und die erhaltene Verbindung gegebenenfalls mit einer Säure in ein Säureadditionssalz überführt, oder daß man

b) eine Verbindung der allgemeinen Formel IV

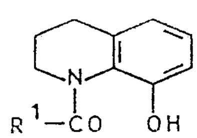

(IV),

in der $R^1$ die angegebene Bedeutung besitzt, mit einer Verbindung der allgemeinen Formel V

$R^2-(CH_2)_n-Y$ (V),

in der $R^2$ und n die angegebenen Bedeutungen besitzen und in der Y eine nucleophil austauschbare Gruppe bedeutet, in an sich bekannter Weise umsetzt und die erhaltene Verbindung gegebenenfalls mit einer Säure in ein Säureadditionssalz überführt.

6. Verfahren nach Anspruch 5, dadurch gekennzeichnet, daß die Reaktion a) in einem organischen Lösungsmittel bei einer Temperatur von 0 bis 160°C, vorzugsweise 20 bis 120°C; die Reaktion b) in einem polaren aprotischen Lösungsmittel in Gegenwart einer starken Base bei einer Temperatur von -40 bis

+100°C, vorzugsweise -20 bis +50°C, oder in einem protischen oder aprotischen polaren Lösungsmittel in Gegenwart einer schwachen bis mittelstarken Base bei einer Temperatur von 0 bis 160°C, vorzugsweise 20 bis 120°C, durchgeführt wird.

7. Pharmazeutische Zubereitung, enthaltend eine wirksame Dosis eines Tetrahydrochinolins der allgemeinen Formel I oder ein pharmazeutisch annehmbares Säureadditionssalz davon, nach einem oder mehreren der Ansprüche 1 bis 4 neben pharmazeutisch zulässigen Träger-und Zusatzstoffen und gegebenenfalls noch einer oder mehreren anderen pharmazeutisch wirksamen Substanzen.

8. Verwendung der Tetrahydrochinoline oder deren pharmazeutisch annehmbare Salze nach einem oder mehreren der Ansprüche 1 bis 4 zur Herstellung von Pharmazeutika auf nicht chemischem Wege.

9. Verwendung der Tetrahydrochinoline oder deren pharmazeutisch annehmbare Salze nach einem oder mehreren der Ansprüche 1 bis 4 zur Herstellung von Antiarrhythmika auf nicht chemischem Wege.

10. Verwendung der Tetrahydrochinoline oder deren pharmazeutisch annehmbare Salze nach einem oder mehreren der Ansprüche 1 bis 4 zur Herstellung von Arzneimitteln zur Behandlung von Erkrankungen des Herz-Kreislauf-Systems, insbesondere von Herzarrhythmien.

Patentansprüche für den folgenden Vertragsstaat: GR

1. Derivate des Tetrahydrochinolins der allgemeinen Formel I

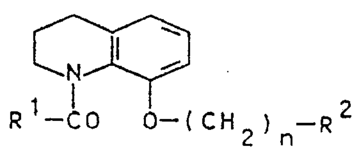

(I)

und deren Säureadditionssalze, wobei
n 2, 3, 4, 5 oder 6,
$R^1$ Alkyl mit 1 bis 5 C-Atomen, Phenyl, durch Alkyl mit 1 bis 3 C-Atomen, Alkoxy mit 1 bis 3 C-Atomen, Halogen oder Trifluormethyl mono-, di-oder trisubstituiertes Phenyl, Thienyl, Furyl, Pyrrolyl oder Pyridyl,
$R^2$ den Rest

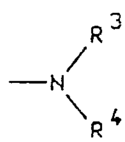

oder

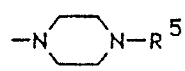

,

$R^3$ und $R^4$ unabhängig voneinander Wasserstoff, Alkyl mit 1 bis 4 C-Atomen, Cycloalkyl mit 3 bis 8 C-Atomen, oder zusammen mit dem Stickstoffatomen, an das sie gebunden sind, 1-Piperidinyl, 4-Morpholinyl, 1-Imidazolidinyl oder 1-Pyrrolidinyl,
$R^5$ Wasserstoff, Alkyl mit 1 bis 5 C-Atomen, Phenyl, Phenylalkyl mit 1 bis 4 C-Atomen in Alkylrest, Phenyloxyalkyl mit 1 bis 4 C-Atomen in Alkylrest, wobei vorhandene Phenylkerne jeweils mit Alkyl mit 1 bis 3 C-Atomen, Alkoxy mit 1 bis 3 C-Atomen, Halogen oder Trifluormethyl mono-, di-oder trisubstituiert sein können, oder Thienyl, Furyl, Pyrrolyl, Pyridinyl, Pyrimidinyl oder Triazinyl bedeuten.

2. Tetrahydrochinolinderivate nach Anspruch 1, dadurch gekennzeichnet, daß n 2, 3 oder 4 bedeutet.

3. Tetrahydrochinolinderivate nach Anspruch 1 und/oder 2, dadurch gekennzeichnet, daß $R^1$ Pyridyl bedeutet.

4. Tetrahydrochinolinderivate nach einem oder mehreren der Ansprüche 1 bis 3, dadurch gekennzeichnet, daß $R^2$ den Rest $-NR^3R^4$ oder den Rest

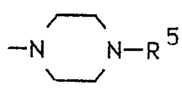

bedeutet, wobei $R^3$ für Wasserstoff, Methyl oder n-Propyl, $R^4$ für n-Propyl, Isopropyl, tert.-Butyl oder Cyclohexyl steht, oder wobei $R^3$ und $R^4$ zusammen mit dem Stickstoffatom, an das sie gebunden sind, 4-Morpholinyl bedeuten und wobei $R^5$ für durch Alkoxy mit 1 bis 3 C-Atomen monosubstituiertes Phenyl steht.

5. Verfahren zur Herstellung der in einem oder mehreren der Ansprüche 1 bis 4 angegebenen Tetrahydrochinolinderivate der Formel I, durch gekennzeichnet, daß man
a) eine Verbindung der allgemeinen Formel II

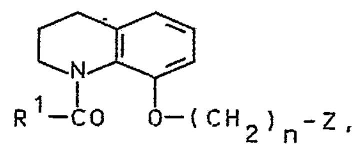
(II)

in der $R^1$ und n die genannten Bedeutungen besitzen und in der Z eine nucleophil austauschbare Gruppe bedeutet, mit einem Amin der allgemeinen Formel III
$R^2$-H (III),
in der $R^2$ die angegebene Bedeutung besitzt, in an sich bekannter Weise umsetzt und die erhaltene Verbindung gegebenenfalls mit einer Säure in ein Säureadditionssalz überführt,-oder daß man
b) eine Verbindung der allgemeinen Formel IV

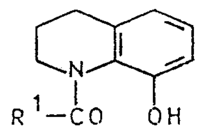
(IV),

in der $R^1$ die angegebene Bedeutung besitzt, mit einer Verbindung der allgemeinen Formel V
$R^2$-$(CH_2)_n$-Y (V),
in der $R^2$ und n die angegebenen Bedeutungen besitzen und in der Y eine nucleophil austauschbare Gruppe bedeutet, in an sich bekannter Weise umsetzt und die erhaltene Verbindung gegebenenfalls mit einer Säure in ein Säureadditionssalz überführt.

6. Verfahren nach Anspruch 5, dadurch gekennzeichnet, daß die Reaktion a) in einem organischen Lösungsmittel bei einer Temperatur von 0 bis 160°C, vorzugsweise 20 bis 120°C; die Reaktion b) in einem polaren aprotischen Lösungsmittel in Gegenwart einer starken Base bei einer Temperatur von -40 bis +100°C, vorzugsweise -20 bis +50°C, oder in einem protischen oder aprotischen polaren Lösungsmittel in Gegenwart einer schwachen bis mittelstarken Base bei einer Temperatur von 0 bis 160°C, vorzugsweise 20 bis 120°C, durchgeführt wird.

7. Verfahren zur Herstellung einer pharmazeutischen Zubereitung, dadurch gekennzeichnet, daß eine oder mehrere Verbindung der allgemeinen Formel I oder ein pharmazeutisch annehmbares Säureadditionssalze davon nach einem oder mehreren der Ansprüche 1 bis 4 durch Mischen mit geeigneten pharmazeutisch zulässigen Träger-und Zusatzstoffen und gegebenenfalls noch einer oder mehreren anderen pharmazeutisch wirksamen Substanzen in eine zur Verabreichung geeignete Form gebracht wird.

8. Verwendung der Tetrahydrochinoline oder deren pharmazeutisch annehmbare Salze nach einem oder mehreren der Ansprüche 1 bis 4 zur Herstellung von Pharmazeutika auf nicht chemischem Wege.

9. Verwendung der Tetrahydrochinoline oder deren pharmazeutisch annehmbare Salze nach einem oder mehreren der Ansprüche 1 bis 4 zur Herstellung von Antiarrhythmika auf nicht chemischem Wege.

10. Verwendung der Tetrahydrochinoline oder deren pharmazeutisch annehmbare Salze nach einem oder mehreren der Ansprüche 1 bis 4 zur Herstellung von Arzneimitteln zur Behandlung von Erkrankungen des Herz-Kreislauf-Systems, insbesondere von Herzarrhythmien.

Patentansprüche für die folgenden Vertragsstaaten: AT, ES

1. Verfahren zur Herstellung von Tetrahydrochinolinen der allgemeinen Formel I

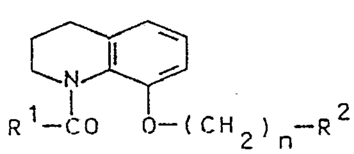
(I)

und deren Säureadditionssalze, wobei

n 2, 3, 4, 5 oder 6,
$R^1$ Alkyl mit 1 bis 5 C-Atomen, Phenyl, durch Alkyl mit 1 bis 3 C-Atomen, Alkoxy mit 1 bis 3 C-Atomen, Halogen oder Trifluormethyl mono-, di-oder trisubstituiertes Phenyl, Thienyl, Furyl, Pyrrolyl oder Pyridyl,
$R^2$ den Rest

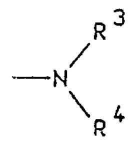

oder

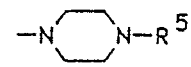

,

$R^3$ und $R^4$ unabhängig voneinander Wasserstoff, Alkyl mit 1 bis 4 C-Atomen, Cycloalkyl mit 3 bis 8 C-Atomen, oder zusammen mit dem Stickstoffatomen, an das sie gebunden sind, 1-Piperidinyl, 4-Morpholinyl, 1-Imidazolidinyl oder 1-Pyrrolidinyl,
$R^5$ Wasserstoff, Alkyl mit 1 bis 5 C-Atomen, Phenyl, Phenylalkyl mit 1 bis 4 C-Atomen in Alkylrest, Phenyloxyalkyl mit 1 bis 4 C-Atomen in Alkylrest, wobei vorhandene Phenylkerne jeweils mit Alkyl mit 1 bis 3 C-Atomen, Alkoxy mit 1 bis 3 C-Atomen, Halogen oder Trifluormethyl mono-, di-oder trisubstituiert sein können, oder Thienyl, Furyl, Pyrrolyl, Pyridinyl, Pyrimidinyl oder Triazinyl bedeuten, dadurch gekennzeichnet, daß man
a) eine Verbindung der allgemeinen Formel II

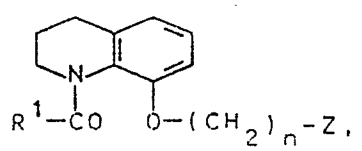

(II)

in der $R^1$ und n die genannten Bedeutungen besitzen und in der Z eine nucleophil austauschbare Gruppe bedeutet, mit einem Amin der allgemeinen Formel III
$R^2$-H (III),
in der $R^2$ die angegebene Bedeutung besitzt, in an sich bekannter Weise umsetzt und die erhaltene Verbindung gegebenenfalls mit einer Säure in ein Säureadditionssalz überführt, oder daß man
b) eine Verbindung der allgemeinen Formel IV

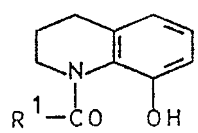

(IV),

in der $R^1$ die angegebene Bedeutung besitzt, mit einer Verbindung der allgemeinen Formel V
$R^2-(CH_2)_n-Y$ (V),
in der $R^2$ und n die angegebenen Bedeutungen besitzen und in der Y eine nucleophil austauschbare Gruppe bedeutet, in an sich bekannter Weise umsetzt und die erhaltene Verbindung gegebenenfalls mit einer Säure in ein Säureadditionssalz überführt.

2. Verfahren nach Anspruch 1, dadurch gekennzeichnet, daß n 2, 3 oder 4 bedeutet.

3. Verfahren nach Ansprüchen 1 und/oder 2, dadurch gekennzeichnet, daß $R^1$ Pyridyl bedeutet.

4. Verfahren nach einem oder mehreren der Ansprüche 1 bis 3, dadurch gekennzeichnet, daß $R^2$ den Rest $-NR^3R^4$ oder den Rest

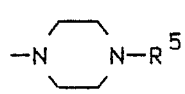

bedeutet, wobei
$R^3$ für Wasserstoff, Methyl oder n-Propyl, $R^4$ für n-Propyl, Isopropyl, tert.-Butyl oder Cyclohexyl steht, oder wobei $R^3$ und $R^4$ zusammen mit dem Stickstoffatom, an das sie gebunden sind, 4-Morpholinyl bedeuten und wobei $R^5$ für durch Alkoxy mit 1 bis 3 C-Atomen monosubstituiertes Phenyl steht.

5. Verfahren nach einem oder mehreren der Ansprüche 1 bis 4, dadurch gekennzeichnet, daß die Reaktion a) in einem organischen Lösungsmittel bei einer Temperatur von 0 bis 160°C, vorzugsweise 20 bis 120°C; die Reaktion b) in einem polaren aprotischen Lösungsmittel in Gegenwart einer starken Base bei einer Temperatur von -40 bis +100°C, vorzugsweise -20 bis +50°C, oder in einem protischen oder aprotischen polaren Lösungsmittel in Gegenwart einer schwachen bis mittelstarken Base bei einer Temperatur von 0 bis 160°C, vorzugsweise 20 bis 120°C, durchgeführt wird.

6. Verwendung der nach einem oder mehreren der Ansprüche 1 bis 5 erhaltenen Tetrahydrochinoline oder deren pharmazeutisch annehmvare Salze zur Herstellung von Pharmazeutika auf nicht chemischem Wege.

7. Verwendung der nach einem oder mehreren der Ansprüche 1 bis 5 erhaltenen Tetrahydrochinoline oder deren pharmazeutisch annehmbare Salze zur Herstellung von Antiarrhythmika auf nicht chemischem Wege.

8. Verwendung der Tetrahydrochinoline oder deren pharmazeutisch annehmbare Salze nach einem oder mehreren der Ansprüche 1 bis 5 zur Herstellung von Arzneimitteln zur Behandlung von Erkrankungen des Herz-Kreislauf-Systems, insbesondere von Herzarrhythmien.

9. Verfahren zur Herstellung einer pharmazeutischen Zubereitung, dadurch gekennzeichnet, daß eine oder mehrere der nach den Ansprüchen 1 bis 5 erhaltenen Verbindungen durch Mischen mit geeigneten pharmazeutisch zulässigen Träger-und Zusatzstoffen und gegebenenfalls noch einer oder mehreren anderen pharmazeutisch wirksamen Substanzen in eine zur Verabreichung geeignete Form gebracht wird.

Europäisches Patentamt

# EUROPÄISCHER RECHERCHENBERICHT

Nummer der Anmeldung

EP 87114549.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A1 - 0 025 864 (CASSELLA)<br>* Ansprüche 1,7 *<br>-- | 1,7-9 | C 07 D 401/06<br>C 07 D 215/26<br>C 07 D 405/06<br>C 07 D 409/06<br>C 07 D 401/14<br>C 07 D 405/14<br>C 07 D 409/14<br>A 61 K 31/47 |
| A | EP - A1 - 0 173 208 (NIHON TOKUSHU)<br>* Formel I *<br>-- | 1 | |
| A | CHEMICAL ABSTRACTS, Band 68, Nr. 1, 1. Jänner 1968, Columbus, Ohio, USA<br>MIKIO HORI, HAJIME FUJIMURA, YUTAKA YAMAKAWA, CHIAKI HARA, KUNIO KITAI; "Benzaza cycloalkane derivatives. II." Seite 262, Spalte 1, Zusammenfassung-Nr. 2796s<br>& Gifu Yakka Daigaku Kiyo No. 16, 61-5(1966)<br>---- | 1 | |

RECHERCHIERTE SACHGEBIETE (Int. Cl.4)

C 07 D 215/00
C 07 D 401/00
C 07 D 405/00
C 07 D 409/00

Der vorliegende Recherchenbericht wurde für alle Patentansprüche erstellt.

| Recherchenort | Abschlußdatum der Recherche | Prüfer |
|---|---|---|
| WIEN | 24-11-1987 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82